(19) Europäisches Patentamt
European Patent Office
Office européen des brevets

(11) EP 0 662 314 B1

(12) **EUROPEAN PATENT SPECIFICATION**

(45) Date of publication and mention of the grant of the patent:
**03.11.1999 Bulletin 1999/44**

(51) Int. Cl.$^6$: **A61K 7/06**, A61K 7/00, A45D 7/06

(21) Application number: **94120018.0**

(22) Date of filing: **16.12.1994**

(54) **Method of hair treatment**

Verfahren zur Behandlung von Haar

Procédé de traitement des cheveux

(84) Designated Contracting States:
**AT DE FR GB NL**

(30) Priority: **16.12.1993 JP 31690693**

(43) Date of publication of application:
**12.07.1995 Bulletin 1995/28**

(73) Proprietor: **Kao Corporation**
**Chuo-Ku Tokyo 103 (JP)**

(72) Inventors:
- **Kure, Naohisa**
  **Tokyo (JP)**
- **Hirano, Yuji**
  **Funabashi-shi, Chiba-ken (JP)**
- **Kajino, Takayoshi**
  **Sumida-ku, Tokyo (JP)**
- **Kondoh, Yukihiro**
  **Utsunomiya-shi, Tochigi-ken (JP)**

(74) Representative:
**Grünecker, Kinkeldey,**
**Stockmair & Schwanhäusser**
**Anwaltssozietät**
**Maximilianstrasse 58**
**80538 München (DE)**

(56) References cited:
**EP-A- 0 411 202**
**EP-A- 0 449 133**
**EP-A- 0 512 270**
**EP-A- 0 529 437**
**DE-A- 4 340 762**
**FR-A- 2 273 492**

- **PATENT ABSTRACTS OF JAPAN vol. 18 no. 511 (C-1253) & JP-A-06 172130 (KAO CORP.) 21 June 1994,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 662 314 B1

## Description

This invention relates to a method of hair treatment for easily and efficiently improving hair quality or repairing damaged hair.

BACKGROUND OF THE INVENTION

Hair treatment is generally conducted in a place where one can wash her hair with warm water by means of a shower (hereinafter sometimes referred to as an "in-bath treatment"); for one thing, one can apply an ample hair treating preparation to the hair and, for another thing, presence of a large quantity of water favors the active ingredient to permeate hairs.
However, an in-bath hair treatment has its own demerits. That is, (1) it cannot be carried out anywhere else. (2) The treating preparation must be finally washed away so that the active ingredient should run out together with water and cannot manifest its effects to the full. (3) For the same reason as in (2), most of the currently available hair treating preparations have almost no effect but leave an improved feel of the hair to the touch. (4) In order to derive the full effect of the active ingredient, a solvent for accelerating permeation should be formulated into the preparation, which would narrow the freedom in designing formulations.
Further, hair treating preparations such as foams and sprays which can be used even in a place other than a bath room (hereinafter sometimes referred to as an "out-bath treatment") are commercially available. Such hair treating preparations have an advantage that they can be used easily in out-bath treatment since they need not be washed away. They have, however, a disadvantage that they dry soon before active ingredients sufficiently permeate into individual hairs, resulting in poor performance of hair treatment.
Other methods of out-bath hair treatment have been proposed wherein the out-bath hair treatment is carried out by using means for generating steam such as a steam dryer, curling irons having means for generating steam and a so-called mist-comb (a comb having means for supplying moisture). These methods have advantages that, since moisture is supplied to hair, hair setting becomes easier and a soft feel is given to hair, while the methods have a disadvantage that such advantages are lost with evaporation of water.
Japanese Patent Laid-open Application 4-367611 discloses curling irons having means for generating steam and integrated with a container. The curling irons disclosed therein can set hair as well as impart gloss, smoothness and scent to hair by simultaneous diffusion of steam and active ingredients. The proposed method has an advantage that volatile ingredients can be effectively applied to hair. It has a disadvantage, however, that nonvolatile ingredients cannot be applied hair. In addition, while active ingredients disclosed therein, for example, volatile silicone, nonionic surfactants and perfume are effective to temporarily impart a soft feel and scent to hair, they are still insufficient for obtaining desired effects of hair treatment, i.e., improvement of hair quality and repair of the damage.

SUMMARY OF THE INVENTION

An object of the present invention is to provide a hair treating preparation which can be used even in an out-bath hair treatment to improve hair quality or repair damaged hair easily, simply, and efficiently.
Another object of the present invention is to provide a method of hair treatment using the above-described hair treating preparation.
The other objects and aspects of the present invention will become apparent from the following description.
As a result of extensive investigations, the present inventors have found that even a nonvolatile hair treating preparation can permeate the inside of hairs without the aid of a permeation accelerator by conducting a steam treatment.
The present invention has been completed based on this finding. The present invention provides a method of hair treatment comprising applying a hair treating preparation to hair and treating the hair with steam either simultaneously with or after the application of the hair treating preparation, in which said hair treating preparation contains a nonvolatile compound as an active ingredient.

DESCRIPTION OF THE PREFERRED EMBODIMENTS

According to a first embodiment of the present invention, the hair treating preparation contains a compound represented by formula (I):

$$R^2-(OCH_2\overset{\overset{\displaystyle R^1}{|}}{C}H)_n-OH \qquad (I)$$

wherein $R^1$ represents a hydrogen atom or a methyl group; $R^2$ represents an alkyl group having 1 to 5 carbon atoms; and n represents an integer of 2 to 6.

The compound represented by formula (I) has no volatility but, when used for hair treatment in combination with steam, produces an effect in preventing hairs from splitting.

If the carbon atom number of $R^2$ exceeds 6, the compound has too large a molecular size to permeate the inside of hairs deeply, failing to exhibit sufficient performance.

The compounds of formula (I) preferably include monoalkyl ethers of diethylene glycol, dipropylene glycol, triethylene glycol or tetraethylene glycol. The alkyl moiety of the monoalkyl ethers preferably contains 1 to 4 carbon atoms.

Specific examples of these compounds include monoalkyl ethers of diethylene glycol or dipropylene glycol (n=2), such as diethylene glycol monomethyl ether, diethylene glycol monoethyl ether, diethylene glycol monopropyl ether, diethylene glycol monobutyl ether, diethylene glycol monopentyl ether, diethylene glycol monoisopropyl ether, diethylene glycol mono-t-butyl ether, dipropylene glycol monomethyl ether, dipropylene glycol monoethyl ether, dipropylene glycol monopropyl ether, dipropylene glycol monobutyl ether, dipropylene glycol monopentyl ether, dipropylene glycol monoisopropyl ether, and dipropylene glycol mono-t-butyl ether; monoalkyl ethers of triethylene glycol (n=3) (the monoalkyl ethers correspond to those enumerated as for diethylene glycol or dipropylene glycol (n=2)); and monoalkyl ethers of tetraethylene glycol (n=4) (the monoalkyl ethers correspond to those enumerated as for diethylene glycol or dipropylene glycol (n=2)).

Preferred compounds are diethylene glycol monoethyl ether, diethylene glycol monopropyl ether, and diethylene glycol monobutyl ether.

The content of the compound of formula (I) in the hair treating preparation of the present invention is not particularly limited but, in general preparation forms, usually ranges from 0.5 to 90% by weight, preferably from 1 to 50% by weight, and still preferably from 2 to 20% by weight. If it is less than 0.5% by weight, the effects as desired cannot be obtained. If it exceeds 90% by weight, no perceptible increase in effects is observed.

The usage or form of the hair treating preparations of the present invention is not restricted as long as the preparation contains the compound of formula (I). For example, the forms of usage include lotions, foams, mists, creams, gels, and sprays, with lotions, mists, and foams being preferred. In conformity with these forms of usage, the compound of formula (I) may be formulated into an aqueous solution, an aqueous alcohol solution, an emulsified cream or a gel.

If desired, and in accordance with the usage or preparation forms, the hair treating preparation of the present invention may contain other components in addition to the compound of formula (I).

For instance, where formulated into a lotion, the preparation preferably contains, as a solvent, water or a mixture of water and a monohydric alcohol having 2 or 3 carbon atoms.

Where formulated into a mist of pumping system, a mixed solvent of water and a monohydric alcohol having 2 or 3 carbon atoms is preferably used as a solvent. Ethanol is particularly preferred as the monohydric $C_{2-3}$ alcohol.

Where formulated into a foam, water or a mixed solvent of water and a monohydric alcohol having 2 or 3 carbon atoms is preferably used as a solvent. Ethanol is particularly preferred as the monohydric $C_{2-3}$ alcohol.

Further, these preparations usually contain a surfactant and a propellant as a foaming agent.

The surfactant to be used as a foaming agent may be cationic, anionic, amphoteric or nonionic, and is preferably nonionic.

Examples of suitable nonionic surfactants include polyoxyalkylene alkyl ethers, such as polyoxyethylene cetyl ether, polyoxyethylene sec-decyl ether, polyoxyethylene stearyl ether, polyoxypropylene cetyl ether, and polyoxyethylene oxypropylene cetyl ether. These surfactants are suitably used in an amount of 0.05 to 1.0% by weight based on the total hair treating preparation.

As propellants, Freon gases, such as trichloromonofluoromethane and dichlorodifluoromethane, may be usable, but liquefied petroleum gas (LPG), dimethyl ether (DME), dimethoxymethane, carbon dioxide, nitrogen gas, or mixtures thereof are preferred. In particular, LPG, DME, dimethoxymethane or a mixture thereof is still preferred. The propellant is used in an amount of 3 to 30% by weight, preferably 5 to 20% by weight, based on the total hair treating preparation.

When formulated into a spray, the preparation preferably contains a monohydric alcohol having 2 or 3 carbon atoms or a mixture of water and a monohydric alcohol having 2 or 3 carbon atoms as a solvent. Ethanol is particularly

preferred as the monohydric $C_{2-3}$ alcohol.

The sprays usually contain a propellant in addition to these solvents.

Any of the above-described gases may be employed as a propellant, with LPG, DME, dimethoxymethane and a mixed gas of LPG, DME and dimethoxymethane being preferred. The propellant is preferably used in an amount of 30 to 95% by weight, still preferably from 40 to 80% by weight, based on the total hair treating preparation.

When formulated into a gel, the preparation preferably contains water or a mixture of water and a lower alcohol or a polyol as a solvent. The lower alcohol preferably includes ethanol and propanol, etc., and the polyol preferably includes propylene glycol and 1,3-butanediol, etc. In addition to the solvent, the gel may contain a thickener comprising a water-soluble polymer, such as polyacrylic acid, hydroxyethyl cellulose, and xanthane gum. The thickener is used in an amount of 0.1 to 5.0% by weight based on the total hair treating preparation.

When formulated into an emulsified cream, the preparation is emulsified by using water as a solvent, a surfactant, and a fat. While the surfactant to be used is not particularly limited, it is preferable to use a cationic surfactant or a combination of a cationic surfactant and a nonionic surfactant. The fat preferably includes amphiphilic ones capable of forming a stable emulsion together with a surfactant. Of amphiphilic fats, higher alcohols are especially preferred.

In addition to the above-mentioned additives necessary for formulating into various forms, the hair treating preparation may contain organic solvents for the purpose of accelerating permeation of the compound of formula (I) into hairs.

Examples of suitable organic solvents are ethanol, isopropyl alcohol, n-propanol, n-butanol, isobutanol, ethylene glycol, diethylene glycol, propylene glycol, dipropylene glycol, 1,3-butanediol, hexylene glycol, benzyl alcohol, cinnamyl alcohol, phenethyl alcohol, p-anisyl alcohol, p-methylbenzyl alcohol, phenoxyethanol, 2-benzyloxyethanol, glycerin, N-methylpyrrolidone, N-octylpyrrolidone, and N-laurylpyrrolidone. Preferred of them are benzyl alcohol, 2-benzyloxyethanol, and N-methylpyrrolidone. The organic solvent is used in an amount of 0.5 to 50% by weight, preferably 1 to 30% by weight, based on the total hair treating preparation.

If desired, the hair treating preparation may furthermore contain various components commonly used in cosmetics as long as the effects of the present invention are not impaired.

While depending on the preparation form, suitable components include cationic, anionic, amphoteric or nonionic surfactants; hydrocarbons, such as liquid paraffin, vaseline, and solid paraffin; lanoline derivatives; higher fatty acids; higher fatty acid esters; animal and vegetable fats and oils, such as mink oil and olive oil; silicone derivatives, such as dimethylpolysiloxane, amino-modified silicone, polyether-modified silicone, alkoxy-modified silicone, and alkyl-modified silicone; cationic, amphoteric, anionic or nonionic polymers; and, as a pH adjusting agent, inorganic alkalis, such as sodium hydroxide, potassium hydroxide, and calcium hydroxide; alcohol amines, such as monoethanolamine and triethanolamine; basic amino acids, such as arginine and lysine; inorganic acids, such as hydrochloric acid; and low-molecular weight carboxylic acids, such as lactic acid and acetic acid.

An ultraviolet absorbent, an antioxidant, an antiseptic, a colorant and a perfume may also be added to the preparation.

The hair treatment according to the first embodiment of the present invention can be carried out by applying the above-mentioned hair treating preparation to hair and treating the hair with steam. The treatment with steam may be either after or simultaneously with the application of the preparation.

In the former case in which application of the hair treating preparation is conducted and thereafter steam treatment is conducted, the hair treating preparation is applied to the whole hair and spread throughout the hair, and after the hair treating preparation get to fit the hair, the hair is treated with steam. While the time between application of the hair treating preparation and stream treatment is not particularly limited, the steam treatment is preferably started within 30 minutes from the application of the hair treating preparation because if the time between them is too long, the hair treating preparation would become too dry for the compound of formula (I) to permeate the inside of the hairs.

In the latter case in which application of the hair treating preparation and steam treatment are conducted simultaneously, the application of the hair treating preparation and the application of steam may be conducted concurrently or alternate with each other. In some types of the hair treating preparation, steam may be generated from the water in which the hair treatment agent is dissolved or mixed.

During the steam treatment, the surface temperature of hairs is preferably kept between 50 and 100°C, still preferably 60 to 90°C. With the surface temperature of hairs being controlled within the above range, the hair treating preparation efficiently penetrates the individual hairs without giving too hot a feel to the scalp.

The degree of permeation of the compound of formula (I) into hairs also depends on the size of steam particles. In the present invention, a recommended vapor size is from 0.5 to 10 μm.

The steam treatment can easily be effected by the use of a commercially available hand steamer. Currently available hand steamers which can preferably be used in the present invention include a hand drier having a steaming mechanism integrally added to a drying mechanism comprising a grip, a heater and a blower, a hand drier having a grooming part, such as a brush or a comb, integrally added to the above-mentioned steaming mechanism and drying mechanism, a curling iron having a steaming mechanism integrally added to a curling mechanism comprising a grip, a

heater and a pair of tongs, and a steaming hot curler comprising a heated type curling rod equipped with a steam generating mechanism.

These hand steamers are commercially sold, for example, under trade names of "Steam Kurukuru", "Ion Treatment", "Steam Ladies Iron", and "Steam Curler", all produced by Matsushita Electric Works, Ltd. The hand steamer which is preferably used in the present invention is also disclosed in Japanese Patent Laid-Open Application 4-367611 which is herein incorporated by reference.

The manner of treating hair with steam by means of these handy type steamers is not particularly restricted. For example, Steam Kurukuru is used in its "steam" mode; Ion Treatment is used in its "steam" mode or "ion steam" mode; and Steam Ladies Iron is used with power on after filling with water. In using Steam Curler, wet a water-retaining part on the surface of a curler with water, set the curler on the heater, and after a prescribed heating time wind hair around the hot curler.

According to a second embodiment of the presentinvention, the hair treating preparation contains a compound represented by formula (II):

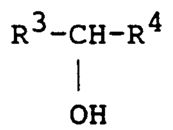

(II)

wherein $R^3$ represents a hydrogen atom or an alkyl group having 1 to 4 carbon atoms; and $R^4$ represents COOH or $SO_3H$, or a salt thereof.

The compound represented by formula (II) has no volatility but, when used for hair treatment using steam, produces an effect of imparting long-lasting softness to the hair.

In the compound represented by formula (II), if the carbon atom number of $R^3$ exceeds 5, the compound has too large a molecular size to permeate the inside of hairs deeply, resulting in insufficient effects.

The compounds of formula (II) include organic acids, such as glycolic acid, lactic acid, α-hydroxybutyric acid and isethionic acid. Salts of these organic acids include sodium salts, potassium salts, calcium salts, ammonium salts, triethanolamine salts, and organic quaternary ammonium salts. The compounds represented by formula (II) and salts thereof may be used either individually or in combination of two or more thereof.

The content of the compound of formula (II) or a salt thereof in the hair treating preparation of the present invention is not particularly limited but, in general preparation forms, usually ranges from 0.5 to 20% by weight, based on the total hair treating preparation. If it is less than 0.5% by weight, the effects as desired cannot be obtained. If it exceeds 20% by weight, no perceptible increase in effects is observed.

The usage and form of the hair treating preparations containing the compound of formula (II) or a salt thereof according to the second embodiment are the same as described with respect to the first embodiment. The other components which may be incorporated into the hair treating preparation of the second embodiment are also the same as those described for the first embodiment.

The hair treatment according to the second embodiment can be carried out using the hair treating preparation containing the compound of formula (II) or a salt thereof in the same manner as in the first embodiment.

According to the third embodiment of the present invention, the hair treating preparation contains at least one compound selected from the group consisting of a naphthalenesulfonic acid, a benzophenonesulfonic acid, an azulenesulfonic acid, and their salts. Although the compounds used in the third embodiment have no volatility, they are capable of imparting long-lasting bouncy and firmness to hair when used together with steam.

The naphthalenesulfonic acid which can be used in the third embodiment includes a compound represented by formula (III):

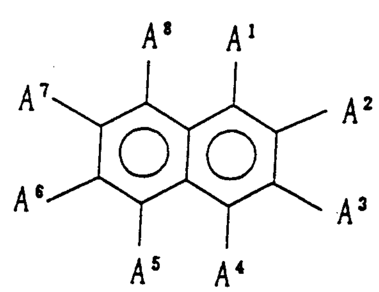

(III)

wherein $A^1$ to $A^8$, which may be the same or different, each represent a sulfo group or a salt thereof, a hydrogen atom, a halogen atom, a hydroxyl group, a nitro group, a carboxyl group, a lower alkoxycarbonyl group, an alkyl group, an alkenyl group, a lower alkoxy group, a formyl group, an acyl group, a substituted or unsubstituted phenylazo group or -NR'R", wherein R' and R", which may be the same or different, each representing a hydrogen atom, a lower alkyl group, a lower alkenyl group, a phenyl group, a benzyl group or an acyl group; and at least one of $A^1$ to $A^8$ is a sulfo group or a salt thereof.

Specific examples of the naphthalenesulfonic acids represented by formula (III) are 1- or 2-naphthalenesulfonic acid, 2,7-naphthalenedisulfonic acid, 1,5-naphthalenedisulfonic acid, 2,6-naphthalenedisulfonic acid, 1,3,6-naphthalenetrisulfonic acid, 1-naphthol-2-sulfonic acid, 1-naphthol-4-sulfonic acid, 2-naphthol-6-sulfonic acid, 2-naphthol-7-sulfonic acid, 1-naphthol-3,6-disulfonic acid, 2-naphthol-3,6-disulfonic acid, 2-naphthol-6,8-disulfonic acid, 2,3-dihydroxynaphthalene-6-sulfonic acid, 1,7-dihydroxynaphthalene-3-sulfonic acid, 4,5-dihydroxynaphthalene-2,7-disulfonic acid (chromotropic acid), 3,6-dihydroxynaphthalene-2,7-disulfonic acid, 1-amino-8-naphthol-4-sulfonic acid, 2-amino-8-naphthol-6-sulfonic acid, 2-amino-5-naphthol-7-sulfonic acid, 1-amino-8-naphthol-3,6-disulfonic acid, 7-amino-1,3-naphthalenedisulfonic acid, 1- amino-2-naphthol-4-sulfonic acid, 1-naphthylamine-4-sulfonic acid, 2-naphthylamine-6-sulfonic acid (Broenner's acid), 1-naphthylamine-7-sulfonic acid (Cleve's acid), 2-naphthylamine-1-sulfonic acid, 1-naphthylamine-6-sulfonic acid, 1-naphthylamine-8-sulfonic acid, 4-amino-5-hydroxy-8-phenylazo-2,7-naphthalenedisulfonic acid, 4-amino-8-(4-carboxyphenylazo)-5-hydroxy-2,7-naphthalenedisulfonic acid, 6-amino-4-hydroxy-3-phenylazo-2-naphthalenesulfonic acid, 4-amino-8-(4-carboxyphenylazo)-5-hydroxy-1-naphthalenesulfonic acid, 7-amino-4-hydroxy-1-phenylazo-2-naphthalenesulfonic acid, 8-amino-5-(4-carboxyphenylazo)-2-naphthalenesulfonic acid, 4-amino-3-(4-carboxyphenylazo)-5-hydroxy-1-naphthalenesulfonic acid, 6-amino-4-hydroxy-5-phenylazo-2-naphthalenesulfonic acid, 2,7-diamino-1-naphthol-3-sulfonic acid, 7,8-diamino-1-naphthol-3-sulfonic acid, a naphthalenesulfonic acid-formalin polycondensate (weight average degree of condensation: 2 to 100), 6-methyl-2-naphthalenesulfonic acid, 4-ethyl-1-naphthalenesulfonic acid, 5-isopropyl-1-naphthalenesulfonic acid, and 5-butyl-2-naphthalenesulfonic acid, as well as salts of these sulfonic acids.

Preferred compounds are 2-naphthalenesulfonic acid, 1,5-naphthalenedisulfonic acid, 2,6-naphthalenedisulfonic acid, 2,7-naphthalenedisulfonic acid, 1-amino-8-naphthol-3,6-disulfonic acid, 1-naphthalenesulfonic acid, chromotropic acid, and a formalin polycondensate of 2-naphthalenesulfonic acid polycondensate (weight average degree of condensation: 2 to 50).

Salts of the naphthalenesulfonic acids include a sodium salt, a potassium salt, a calcium salt, an ammonium salt and an organic quaternary ammonium salt.

The benzophenonesulfonic acids which can be used in the third embodiment include compounds represented by formula (IV):

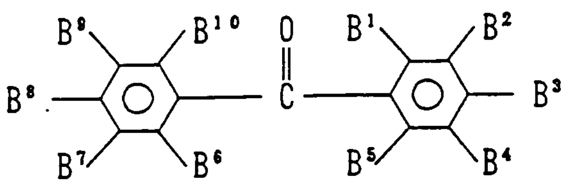

(IV)

wherein $B^1$ to $B^{10}$, which may be the same or different, each representing a sulfo group or a salt thereof, a hydrogen atom, a halogen atom, a hydroxyl group, a carboxyl group, a lower alkyl group, a lower alkenylgroup, a lower alkoxy group or an acyl group; and at least one of $B^1$ to $B^{10}$ is a sulfo group or a salt thereof.

Specific examples of the benzophenonesulfonic acids represented by formula (IV) are hydroxymethoxybenzophenonesulfonic acid, dihydroxymethoxybenzophenonesulfonic acid, dihydroxydimethoxybenzophenonesulfonic acid, dihydroxydi oxybenzophenonesulfonic acid, dihydroxydimethoxybenzophenonedisulfonic acid, o-chlorobenzophenonesulfonic acid, p-chlorobenzophenonesulfonic acid, 4,4'-dichlorobenzophenonesulfonic acid, 2,4'-dichlorobenzophenonesulfonic acid, 2,4-dichlorobenzophenonesulfonic acid, 2-hydroxybenzophenonesulfonic acid, 4-hydroxybenzophenonesulfonic acid, 2,4-dihydroxybenzophenonesulfonic acid, 2,2',4,4'-tetrahydroxybenzophenonesulfonic acid, 2-aminobenzophenonesulfonic acid, 4-aminobenzophenoneuslfonic acid, 4,4'-diaminobenzophenonesulfonic acid, 2-methylbenzophenonesulfonic acid, 4-methoxybenzophenonesulfonic acid, 4,4'-dimethylbenzophenonesulfonic acid, 4,4'-dimethoxybenzophenonesulfonic acid, 4-chloro-4'-hydroxybenzophenonesulfonic acid, 2-chloro-5-hydroxybenzophenonesulfonic acid, and 2-hydroxy-4-methoxy-3'-benzophenone-5-sulfonic acid.

Preferred compounds are hydroxymethoxybenzophenonesulfonic acids, such as 2-hydroxy-4-methoxybenzophenone-5-sulfonic acid and 2-hydroxy-4-methoxybenzophenone-3-sulfonic acid; dihydroxymethoxybenzophenonesulfonic acids, such as 2,2'-dihydroxy-4-methoxybenzophenone-3-sulfonic acid and 2,2'-dihydroxy-4-methoxybenzophenone-5-sulfonic acid; dihydroxydimethoxybenzophenonesulfonic acids, such as 2,2'-dihydroxy-4,4'-dimethoxybenzophenone-3-sulfonic acid and 2,2'-dihydroxy-4,4'-dimethoxybenzophenone-5-sulfonic acid; and dihydroxydimethoxybenzophenonedisulfonic acids, such as 2,2'-dihydroxy-4,4'-dimethoxybenzophenone-3,3'-disulfonic acid, 2,2'-dihydroxy-4,4'-dimethoxybenzophenone-5,5'-disulfonic acid, and 2,2'-dihydroxy-4,4'-dimethoxybenzophenone-3,5'-disulfonic acid.

Salts of the benzophenonesulfonic acids include sodium salt, a potassium salt, a lithium salt, a calcium salt, an ammonium salt, and an organic quaternary ammonium salt.

The azulenesulfonic acids which can be used in the third embodiment include guaiazulenesulfonic acid (i.e., 7- isopropyl-1,4-dimethyl-3-azulenesulfonic acid), ethyl guaiazulenesulfonate, 1-azulenesulfonic acid, 3-acetyl-7-isopropyl-1-azulenesulfonic acid, 3-(2-hydroxyethyl)-7-isopropyl-1-azulenesulfonic acid, 3-methyl-7-isopropyl-1-azulenesulfonic acid, 7-isopropyl-1-azulenesulfonic acid, 3-phenyl-6-isopropyl-1-azulenesulfonic acid, 1,4-dimethyl-7-isopropyl-2-azulenesulfonic acid, 4-ethoxy-3-ethyl-6-isopropyl-1-azulenesulfonic acid, 1,3-azulenedisulfonic acid, 4,6,8-trimethyl-1,3-azulenedisulfonic acid, 1,3-bis(1,1-dimethylethyl)-5,7-azulenedisulfonic acid, 1,3-bis(1,1-dimethylethyl)-5-azulenesulfonic acid, and 3-formyl-4,6,8-trimethyl-1-azulenesulfonic acid.

Preferred compounds are 4-ethoxy-3-ethyl-6-isopropyl-1-azulenesulfonic acid, 1,4-dimethyl-7-isopropyl-2-azulenesulfonic acid, guaiazulenesulfonic acid, and ethyl guaiazulenesulfonate.

Salts of the azulenesulfonic acid include a sodium salt, a potassium salt, a calcium salt, an ammonium salt, and an organic quaternary ammonium salt.

The above-mentioned naphthalenesulfonic acids, benzophenonesulfonic acids, and azulenesulfonic acids, and salts thereof may be used either individually or in combination of two or more thereof.

The content of the compound selected from the group consisting of naphthalenesulfonic acids, benzophenonesulfonic acids, and azulenesulfonic acids, and their salts in the hair treating preparation of the present invention is not particularly limited but, in general preparation forms, usually ranges from 0.1 to 10% by weight, based on the total hair treating preparation. If it is less than 0.1% by weight, the effects as desired cannot be obtained. If it exceeds 10% by weight, no particular increase in effects results.

The usage and form of the hair treating preparations according to the third embodiment are the same as described with respect to the first embodiment.The other components which may be incorporated into the hair treating preparation of the third embodiment are also the same as those described for the first embodiment.

The hair treatment according to the third embodiment can be carried out using the hair treating preparation containing the aforesaid compound in the same manner as in the first embodiment.

According to the fourth embodiment of the present invention, the hair treating preparation comprises at least one nonionic amphiphilic compound which comprises at least one long-chain branched alkyl group or alkenyl group in the molecule thereof and has an HLB of from 2 to 12, and said nonionic amphiphilic compound or the mixture of said nonionic amphiphilic compound and water maintains a liquid crystal structure at any temperature between 0 and 50°C.

The nonionic amphiphilic compound used in the forth embodiment has no volatility but, when used for hair treatment in combination with steam, produces an effect of improving moistness and smoothness of hair.

The nonionic amphiphilic compound contains at least one nonionic amphiphilic compound containing at least one long-chain branched alkyl group or alkenyl group in the molecule thereof and having an HLB of from 2 to 12. If the HLB is less than 2, the hair treating preparation becomes oily. If it exceeds 12, the hair treating preparation has a reduced conditioning effect. A preferred HLB is from 3 to 10.

The term "HLB" (hydrophile lipophile balance) as used herein means a value calculated according to the following formula suggested by Oda, Teramura, et. al.

$$HLB = (\Sigma\text{inorganic value}/\Sigma\text{organic value}) \times 10$$

The nonionic amphiphilic compound itself or a mixture of the nonionic amphiphilic compound and water maintains a liquid crystal structure at any temperature between 0 and 50°C, preferably between 5 and 40°C, still preferably between 5 and 35°C. The term "the nonionic amphiphilic compound itself or mixture of the nonionic amphiphilic compound water is capable of forming a liquid crystal structure at at any temperature" as used herein means the nonionic amphiphilic compound itself or a mixture of the nonionic amphiphilic compound water is capable of forming a liquid crystal structure at at least one point within the temperature range.

The details for the above-mentioned nonionic amphiphilic compound are described in Japanese Patent Laid-Open Application 5-124921.

While the liquid crystal structure formed depends on the nonionic amphiphilic compound to be used, it may include a lamella phase and a reversed middle phase.

A lamella phase of a nonionic amphiphilic compound can be confirmed by X-ray diffractometry and measurement with a differential scanning calorimeter (DSC) in accordance with the method described in The Journal of Cell Biology, Vol. 12, pp. 207-209 or "Hyomen", Vol. 11, No. 10, pp. 579-590.

Nonionic amphiphilic compounds capable of maintaining a lamella liquid crystal structure include the following

compounds (i) to (iv).

(i) Glycerylated polyol represented by formula (V):

$$A_{\underline{a}}(G) \qquad (V)$$

wherein G represents a residual group obtained by removing a hydroxyl group(s) from a polyol selected from the group consisting of pentaerythritol, sorbitol, maltitol, glycol, fructose, and an alkyl glycoside; A represents

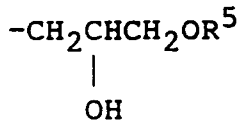 or 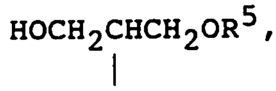,

wherein $R^5$ represents a branched alkyl or alkenyl group having 10 to 36 carbon atoms; and a represents an integer of not less than 1 and not more than the total number of the hydroxyl groups of the polyol; when a is 2 or more, the plural A groups may be the same or different.

(ii) Methyl-branched fatty acid esters represented by formula (VI):

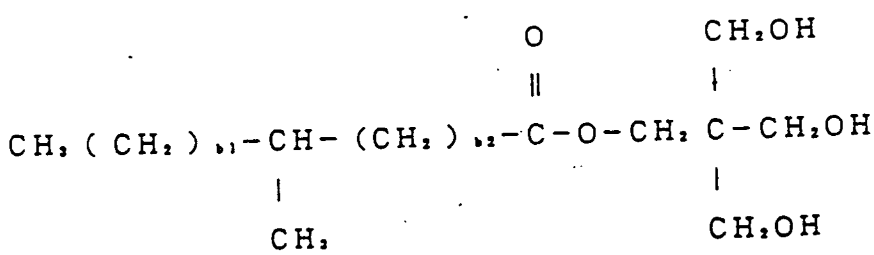

(VI)

wherein $b_1$ and $b_2$, which may be the same or different, each representing 0 or an integer of from 1 to 33, provided that the sum of $b_1$ and $b_2$ is from 6 to 33.

(iii) Branched fatty acid glyceroglycolipids represented by formula (VII):

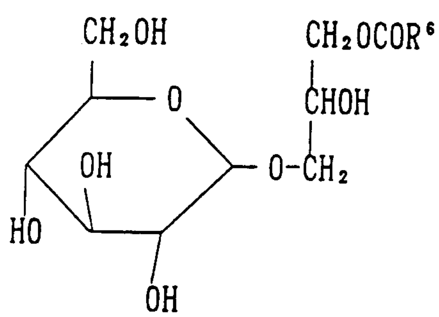

(VII)

wherein $R^6$ represents

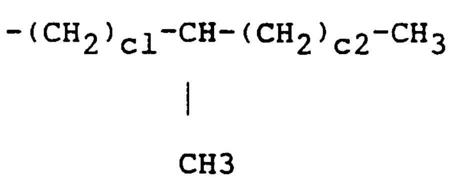 or 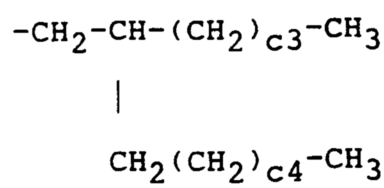

wherein $c_1$ and $c_2$, which may be the same or different, each represent an integer of from 0 to 33, provided that the sum of $c_1$ and $c_2$ is from 6 to 33; and $c_3$ and $c_4$, which may be the same or different, each represent 0 or an integer

of from 0 to 31, provided that the sum of $c_3$ and $c_4$ is from 4 to 31.

(iv) Alkyltrismethylols represented by formula (VIII) or alkyltrismethylolamides represented by formula (IX):

$$R^7\text{-}C(CH_2OH)_3 \qquad (VIII)$$

$$R^7\text{-}CONHC(CH_2OH)_3 \qquad (IX)$$

wherein $R^7$ represents a straight-chain or branched alkyl group having 6 to 22 carbon atoms, preferably methyl-branched isostearyl group.

In formula (V) representing glycerylated polyols (i), the alkyl glycoside represented by G includes methyl glucoside, ethyl glucoside, propyl glucoside, octyl glucoside, methyl maltoside, and ethyl maltoside. $R^5$ preferably represents a branched alkyl group having 10 to 36 carbon atoms, preferably 16 to 36 carbon atoms, still preferably 18 to 24 carbon atoms. Such a branched alkyl group preferably includes those represented by the following formulae:

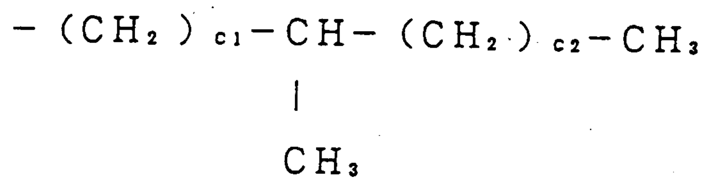

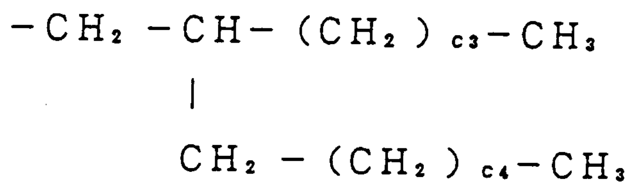

wherein $c_1$, $c_2$, $c_3$, and $c_4$ are as defined above.

Specific examples of suitable branched alkyl groups as $R^5$ are methylpentadecyl, methylhexadecyl, methylheptadecyl (isostearyl), methyloctadecyl, methylbehenyl, ethylhexadecyl, ethyloctadecyl, ethylbehenyl, butyldodecyl, butylhexadecyl, butyloctadecyl, hexyldecyl, heptylundecyl, octyldodecyl, decyldodecyl, decyltetradecyl, dodecylhexadecyl, and tetradecyloctadecyl groups.

<u>a</u> in formula (V) is preferably 1 or 2.

The alkenyl group as represented by $R^5$ preferably includes a palmitoleyl, oleyl, and docosenyl groups.

The glycerylated polyol (i) can be prepared by, for example, reacting a polyol with a branched alkyl glycidyl ether in the presence of a basic catalyst according to the following reaction formula:

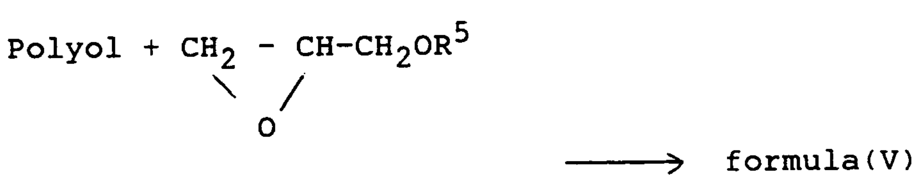


The molar ratio of the polyol and the branched alkyl glycidyl ether is selected appropriately according to a desired degree of etherification of the resulting glycerylated polyol.

The glycerylated polyol as produced by the above reaction is usually a mixture of an adduct formed by addition of one mole of a branched alkyl glycidyl ether per mole of a polyol (hereinafter referred to as a 1-mole adduct), an adduct formed by addition of 2 moles of a branched alkyl glycidyl ether per mole of a polyol (hereinafter referred to as a 2-mole adduct), and an adduct formed by addition of 3 or more moles of a branched alkyl glycidyl ether per mole of a polyol (hereinafter referred to as a poly-mole adduct). In order to obtain a glycerylated polyol having a high proportion of a 1-mole adduct, it is preferable to use the polyol in excess at a molar ratio of the polyol to the branched alkyl glycidyl ether usually ranging from 1.2: 1.0 to 10.0 to 1.0. Taking the yield of the 1-mole adduct and recovery of the excess polyol into consideration, a preferred polyol to branched alkyl glycidyl ether molar ratio is from 1.5:1.0 to 5.0:1.0. In order to

obtain a glycerylated polyol having a high proportion of a 2-mole adduct, it is preferable to use the branched alkyl glycidyl ether in excess at a molar ratio of the polyol to the branched alkyl glycidyl ether usually ranging from 0.3:1.0 to 1.1:1.0. Taking the yield of the 2-mole adduct into consideration, a preferred polyol to branched alkyl glycidyl ether molar ratio is from 0.4:1.0 to 0.8:1.0.

While the above reaction is usually conducted without a solvent, use of an organic solvent is recommended for facilitating mixing of the polyol and the branched alkyl glycidyl ether. Suitable organic solvents include dimethyl sulfoxide, dimethylacetamide, dimethylformamide, and N-methylpyrrolidone. The organic solvent is preferably used in an amount of 0.1 to 10.0 times the volume of the polyol.

As a catalyst used in the above reaction, an acid or a base catalyst, which is commonly known as a catalyst used for reaction of a epoxy group, can be used. However, an acid catalyst is not preferred because the presence of an acid catalyst sometimes causes such such side reactions as decomposition of the ether linkage of the resulting glycerlated polyol or dehydration of a hydroxyl group. Therefore, it is prefarable to use a basic catalyst which is free from such side reactions.

The basic catalysts to be used are not particularly limited. From the standpoint of reactivity and economy, sodium hydroxide, potassium hydroxide, sodium methylate, sodium ethylate, and sodium hydride are preferred. The basic catalyst is preferably used in an amount of 0.01 to 20% by weight, still preferably 0.1 to 10.0% by weight, based on the polyol.

The reaction is preferably performed at a temperature of from 50 to 200°C, still preferably from 80 to 150°C. At temperatures lower than 50°C, the reaction rate is low. At temperatures higher than 200°C, the product will be colored.

In the above reaction, water is preferably removed from the reaction system before addition of a branched alkyl glycidyl ether for reaction because the presence of water in the reaction system causes the epoxy group of the branched alkyl glycidyl ether to react with water to produce a glyceryl. The reaction system can be freed of water by bubbling dry nitrogen gas through a heated solution or dispersion of a polyol in a organic solvent or dehydrating the polyol solution or dispersion by heating under reduced pressure.

After completion of the reaction, an organic acid (e.g., acetic acid or citric acid) or an inorganic acid (e.g., sulfuric acid, hydrochloric acid or phosphoric acid) is added to the reaction system toneutralize the catalyst, and the organic solvent is removed. Removal of the solvent is preferably conducted under reduced pressure usually at 120°C or lower in order to avoid thermal decomposition of the reaction product.

As stated above, the glycerylated polyol as produced by the above reaction is usually a mixture of a 1-mole adduct, a 2-mole adduct, and a poly-mole adduct other than the 1-mole adduct, the 2-mole adduct and the poly-mole adduct. The mixture may be used as such as a glycerylated polyol of the fourth embodiment. If desired, the mixture may be separated into each component by known means, such as column chromatography on silica gel or solvent extraction, to use, for example, the 1-mole adduct only.

The glycerylated polyol as produced by the above reaction sometimes contains an unreacted glycoside other than the 1-mole adduct, the 2-mole adduct and the poly-mole adduct. Unless any practical problem occurs, such a glycerylated polyol containing the unreacted glycoside may be used under the state where unreacted glycoside is contained. If desired, the unreacted glycoside may be removed by any known purification method, such as a method of using a two-phase extracting solvent system using such organic solvents as ethyl acetate, methyl ethyl ketone, methyl isobutyl ketone chloroform, and Smith film distillation.

In formula (VI) representing methyl-branched fatty acid esters (ii), the sum of $b_1$ and $b_2$ is from 6 to 33. From the viewpoint of performance as a cosmetic material, the sum of $b_1$ and $b_2$ is preferably from 10 to 16, still preferably 14. The methyl branch is preferably near the center of the alkyl main chain.

The methyl-branched fatty acid ester of formula (VI) can be obtained by reacting a lower alkyl ester of a methyl-branched fatty acid with pentaerythritol according to the following reaction formula:

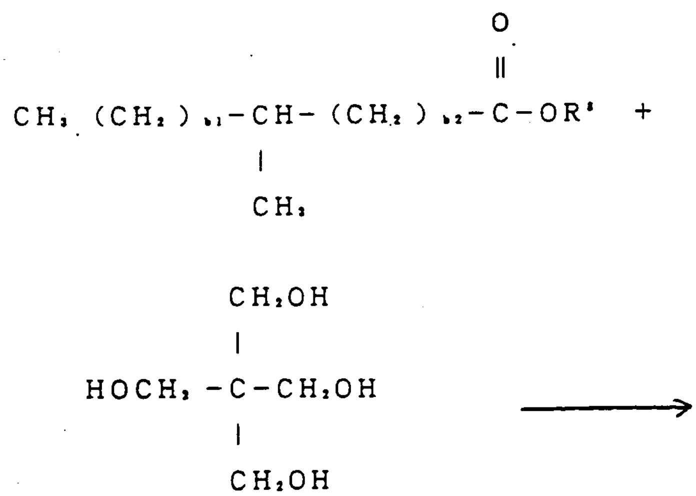

formula (VI)

wherein $b_1$ and $b_2$ are as defined above; and $R^8$ represents an alkyl group having 1 to 18 carbon atoms, preferably 1 to 3 carbon atoms.

The lower alkyl ester of a methyl-branched fatty acid is obtained by esterifying the corresponding carboxylic acid in a conventional manner. Of the corresponding carboxylic acids, those obtainable on an industrial scale are usually mixtures in which the total carbon atom number of the alkyl group and the position of the methyl branch each has a given distribution. For example, the mixed carboxylic acid obtained as a by-product in the production of an oleic acid dimer comprises about 75% or more of isostearic acid having a total carbon atom number of 18 (the sum of $b_1$ and $b_2$ being 14), with the rest comprising other carboxylic acids having a total carbon atom number of 14, 16 and 20, in which the methyl branch being positioned almost in the middle of the alkyl main chain (see J. Amer. Oil Chem. Soc., Vol. 51, p. 522 (1974)).

In the above reaction, a molar ratio of the lower alkyl ester of a methyl-branched fatty acid and pentaerythritol is preferably from 1/1 to 10/1.

While the solvent to be used in the reaction is not particularly limited, it preferably selected from those capable of dissolving both the lower alkyl ester of a methyl-branched fatty acid and pentaerythritol. For example, dimethylformamide is suitably used.

The above reaction is usually carried out in the presence of an alkali catalyst, preferably sodium methylate. While the catalyst is not limited, it is preferably used in an amount of 0.1 to 20 mol% based on the methyl-branched fatty acid lower alkyl ester.

The reaction temperature is selected from the range of from 60 to 150°C.

The produced methyl-branched fatty acid ester can be isolated from the reaction mixture in a usual manner, for example, distillation, recrystallization, chromatography, or a combination thereof.

In formula (VII) representing branched fatty acid glyceroglycolipids (iii), the sum of $c_1$ and $c_2$ in $R^7$ is from 6 to 36. From the standpoint of performance as a cosmetic material, the sum of $c_1$ and $c_2$ is preferably 10 to 16, still preferably 14, similarly to $b_1$ and $b_2$. Likewise, while the sum of $c_3$ and $c_4$ ranges from 4 to 31, it is preferably 6 to 14, still preferably 8 to 12.

The branched fatty acid glyceroglycolipid (iii) can be obtained by, for example, reacting compound (7a) with fatty acid (7b) according to the following formula:

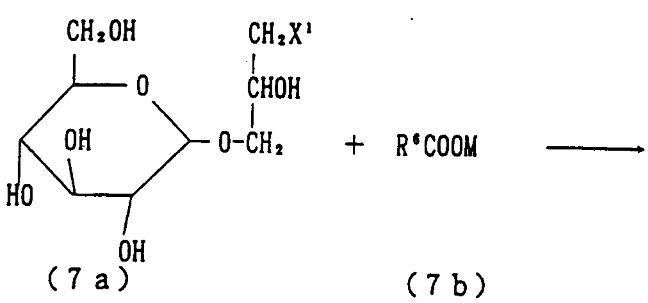

(7a) (7b)

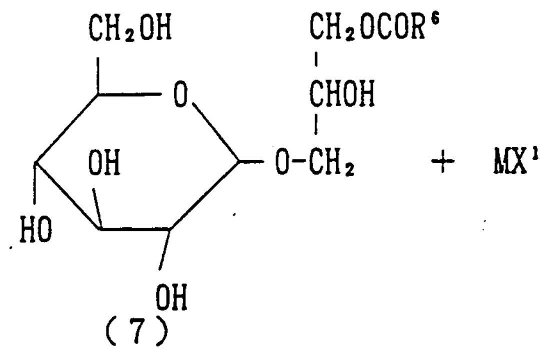

(7)

wherein $X^1$ represents a halogen atom; M represents a hydrogen atom or a cation; and $R^6$ is as defined above.

Compound (7a) can easily be prepared by a known process, for example, by reacting a monosaccharide or an oligosaccharide with a glycerol monohalohydrin, a glycerol dihalohydrin, or an epihalohydrin.

Compound (7b) can be obtained by, for example, reacting a fatty acid with an alkali metal hydroxide (e.g., sodium hydroxide) or an amine in an appropriate solvent. The cation as represented by M includes an alkali metal, an ammonium group, an alkylammonium group, and a trialkanolamine.

The above reaction can be carried out by allowing compounds (7a) and (7b) to react at 30 to 150°C, preferably 70 to 120°C. Compound (7b) is usually used in an amount of 0.3 to 3.0 moles, preferably 1.0 to 2.0 moles, per mole of compound (7a). Where M in compound (7b) is a hydrogen atom, the reaction is conducted in the presence of an alkaline substance, such as an alkali metal hydroxide (e.g., sodium hydroxide or potassium hydroxide), an alkali metal alcoholate, or an alkylamine hydroxide.

For facilitating mixing of compounds (7a) and (7b) and for smoothening the reaction, a polar solvent may be used in the reaction. Examples of suitable polar solvents include dimethylformamide, dimethylacetamide, dimethyl sulfoxide, N-methylpyrrolidone, pyridine, water, and a mixture thereof. The amount of the polar solvent to be used is determined appropriately.

If desired, a phase transfer catalyst may be used. The amount of a phase transfer catalyst to be used is appropriately selected usually from the range of from 0.1 to 10 mol% based on compound (7b).

Suitable phase transfer catalysts include tetraalkylammonium halides, such as tetraethylammonium bromide, tetrapropylammonium bromide, tetrabutylammonium bromide, tetraheptylammonium bromide, tetrahexylammonium bromide, N,N,N-trimethyl-N-octylammonium chloride, N,N,N-trimethyl-N-decylammonium chloride, N,N,N-trimethyl-N-dodecylammonium chloride, N,N,N-trimethyl-N-hexadecylammonium chloride, N,N,N-trimethyl-N-octadecylammonium chloride, N,N-dimethyl-N,N-dihexadecylammonium chloride, and N,N-dimethyl-N,N-dioctadecylammonium chloride.

The product as obtained usually contains by-products, such as an inorganic salt, and unreacted compounds (7a) and/or (7b) in addition to the desired glyceroglycolipid. Depending on the final use, the product containing such by-products or unreacted compounds may be used as it is. Where more purified material is necessary, it may be purified appropriately by any known methods, such as partitioning chromatography, adsorption chromatography, solvent fractionation, recrystallization, and the like.

The nonionic amphiphilic compounds (i) to (iv) are thermotropic liquid crystal compounds which are capable of maintaining a lamella liquid crystal structure by themselves at any temperature within a range of from 0°C to 50°C. They also has such an advantage that they are dispersed in water substantially uniformly as lamellar liquid crystals. A suitable mixing ratio of water to the nonionic amphiphilic compound for forming such a liquid crystal structure ranges from 99/1 to 1/99 by weight.

On the other hand, nonionic amphiphilic compounds capable of exhibiting a reversed middle phase include α-mono(methyl-branched alkyl) glyceryl ether represented by formula (X):

$$R^9\text{-}OCH_2CH(OH)CH_2OH \qquad (X)$$

wherein $R^9$ represents a methyl-branched alkyl group having 9 to 36 carbon atoms.

Suitable methyl-branched alkyl group as $R^9$ include those having formula:

$$CH_3(CH_2)_m-\underset{\displaystyle CH_3}{\underset{|}{CH}}-(CH_2)_n-$$

wherein m and n, which may be the same or different, each represent an integer of 0 to 33, provided that the sum of m and n is from 6 to 33.

A particularly preferred methyl-branched alkyl group is an isostearyl group.

The α-mono(methyl-branched alkyl) glyceryl ether is prepared in accordance with the method described in Japanese Patent Publications 61-26997 and 62-1368. The α-mono(methyl-branched alkyl) glyceryl ether forms a uniform reversed middle liquid crystal structure upon being mixed with water at any temperature within a range of from 0 to 50°C. A suitable mixing ratio of water to the α-mono(methyl-branched alkyl) glyceryl ether for formation of a reversed middle liquid crystal structure ranges from 99/1 to 1/99 by weight.

The hair treating preparation of the forth embodiment may contain one or more than one of the above-mentioned nonionic amphiphilic compounds.

The content of the nonionic amphiphilic compound in the hair treating preparation of the present invention is not particularly limited but, in general preparation forms, usually ranges from 0.01 to 30% by weight, preferably from 0.05 to 20% by weight. If it is less than 0.05% by weight, the effects as desired cannot be obtained. If it exceeds 20% by weight, the resulting hair treating preparation feels sticky.

The usage and form of the hair treating preparations comprising nonionic amphilic compounds according to the fourth embodiment are the same as described with respect to the first embodiment. The other components which may be incorporated into the hair treating preparation according to the fourth embodiment are also the same as those described for the first embodiment.

The hair treatment according to the fourth embodiment can be carried out using the hair treating preparation containing the aforesaid nonionic amphiphilic compound in the same manner as in the first embodiment.

The aforesaid first to fourth embodiments of the present invention may be combined appropriately. That is, the compounds used in the first to fourth embodiments may be used in combination to obtain a complex effect of their several effects.

According to the present invention, hair treatment for improvement of hair quality and repair of damaged hair can be carried out easily, conveniently and efficiently at any place as well as in a bathroom.

The first embodiment is particularly effective to prevent hair from splitting; the second embodiment is particularly effective to impart improved and long-lasting softness to hair; the third embodiment is particularly effective to impart improved and long-lasting bouncy and firmness to hair; and the fourth embodiment is particularly effective to impart improved and long-lasting moistness and smoothness to hair.

The present invention will be understood more readily with reference to the following Examples. However, these Examples are to illustrate the invention but are not to be construed to limit the scope of the invention.

In the Examples, all the percents are by weight unless otherwise indicated.

EXAMPLE 1

Hair of a Japanese with no history of cold permanent waving or bleaching was shampooed twice and dried, and a bundle of 1000 hairs was prepared therefrom.

A hair lotion was prepared by mixing 15.0% of diethylene glycol monoethyl ether, 35.0% of ethanol, and 50.0% of purified water. The lotion was applied to the hair in an amount of 2 g per bundle and thoroughly spread throughout the bundle.

The hair was combed for 5 minutes while carefully applying steam all over the bundle by the use of a commercially available hand steamer Ion Treatment.

The thus treated hair bundle was allowed to stand at 20°C and 65% RH for 24 hours for spontaneous drying and then given 5000 brushes. The number of split hairs which were produced by the brushing was counted and graded according to the following standard. The result obtained is shown in Table 1.

EXAMPLE 2

The same procedure as in Example 1 was repeated, except for replacing diethylene glycol monoethyl ether with diethylene glycol monopropyl ether. The result obtained is shown in Table 1.

EXAMPLE 3

The same procedure as in Example 1 was repeated, except for replacing diethylene glycol monoethyl ether with diethylene glycol monopropyl ether and replacing Ion Treatment (a hand steamer) with another commercially available hand steamer Steam Ladies Iron. The result obtained is shown in Table 1.

COMPARATIVE EXAMPLE 1

The same procedure as in Example 1 was repeated, except that steam was not applied to the hair. The result obtained is shown in Table 1.

COMPARATIVE EXAMPLE 2

The same procedure as in Example 2 was repeated, except that steam was not applied to the hair. The result obtained is shown in Table 1.

COMPARATIVE EXAMPLE 3

The same procedure as in Example 3 was repeated, except that steam was not applied to the hair. The result obtained is shown in Table 1.

COMPARATIVE EXAMPLE 4

The same procedure as in Example 1 was repeated, except that the lotion was not applied to the hair. The result obtained is shown in Table 1.

TABLE 1

| | | Example | | | Compara. Example | | | |
|---|---|---|---|---|---|---|---|---|
| | | 1 | 2 | 3 | 1 | 2 | 3 | 4 |
| Hand steamer * | | A | A | B | none | none | none | A |
| Composition of Lotion (%) | Diethylene glycol monoethyl ether | 15.0 | -- | -- | 1 5.0 | -- | -- | not applied |
| | Diethylene glycol monopropyl ether | -- | 15.0 | -- | -- | 15.0 | -- | |
| | Diethylene glycol monobutyl ether | -- | -- | 15.0 | -- | -- | 15.0 | |
| | Ethanol | 35.0 | 35.0 | 35.0 | 35.0 | 35.0 | 35.0 | |
| | Purified water | 50.0 | 50.0 | 50.0 | 50.0 | 50.0 | 50.0 | |
| Hair Splitting | | ○ | ○ | ○ | X | X | X | X |

Note: *:
A: Ion Treatment
B: Steam Ladies Iron
Standard of Evaluation:
◎ The number of split hairs was 20 or less.
○ The number of split hairs was 21 to 50.
△ The number of split hairs was 51 to 100.
X The number of split hairs was 101 or more.

As is apparent from the results in Table 1, the hair treated by the method of the present invention has a reduced occurrence of hair splitting.

EXAMPLE 4

Hair of a Japanese was shampooed twice and dried, and a bundle weighing 5 g was prepared therefrom.

A hair foam was prepared from 10.0% of glycolic acid, 1.0% of Softanol 90 (polyoxyethylene-sec-tetradecyl ether (9E.O.), a foaming agent produced by Nippon Shokubai Kagaku Kogyo Co., Ltd.), an adequate amount of sodium hydroxide, 7.0% of LPG, and purified water (the balance). Two grams of the foam were applied to the hair bundle and thoroughly spread throughout the bundle, and the hair was combed for 5 minutes while carefully applying steam all over the bundle by the use of a commercially available hand steamer Ion Treatment.

The thus treated hair bundle was allowed to stand at 20 °C and 65% RH for 12 hours and then given a shampoo, followed by drying with a dryer. The softness of the hair bundle was organoleptically examined after 3 or 12 hours' standing at 20°C and 65% RH and after 12 hours' standing and a shampoo and drying and evaluated in comparison with a hair bundle having been treated with neither the foam nor steam. The standard of evaluation is shown below. The results obtained are shown in Table 2.

EXAMPLE 5

The same procedure as in Example 4 was repeated, except for replacing glycolic acid with 13.7% of α-hydroxybutyric acid (equivalent in mole to 10.0% of glycolic acid) and replacing Ion Treatment with another commercially available hand steamer Steam Ladies Iron. The results of the organoleptic test are shown in Table 2.

EXAMPLE 6

The same procedure as in Example 4 was repeated, except for replacing glycolic acid with 16.6% of isethionic acid (equivalent in mole to 10.0% of glycolic acid) and replacing Ion Treatment with Steam Ladies Iron. The results of the organoleptic test are shown in Table 2.

COMPARATIVE EXAMPLE 5

The same procedure as in Example 4 was repeated, except that steam was not applied to the hair. The results obtained is shown in Table 2.

COMPARATIVE EXAMPLE 6

The same procedure as in Example 5 was repeated, except that steam was not applied to the hair. The results obtained is shown in Table 2.

COMPARATIVE EXAMPLE 7

The same procedure as in Example 6 was repeated, except that steam was not applied to the hair. The results obtained is shown in Table 2.

COMPARATIVE EXAMPLE 8

The same procedure as in Example 5 was repeated, except that the foam was not applied to the hair. The results obtained is shown in Table 2.

TABLE 2

| | | Example | | | Compara. Example | | | |
|---|---|---|---|---|---|---|---|---|
| | | 4 | 5 | 6 | 5 | 6 | 7 | 8 |
| Hand steamer * | | A | B | B | None | None | None | B |
| Composition of Foam (%) | Glycolic acid | 10.0 | – | – | 10.0 | – | – | not applied |
| | α-Hydroxybutyric acid | – | 13.7** | – | – | 13.7** | – | |
| | Isethionic acid | – | – | 16.6** | – | – | 16.6** | |
| | Softanol 90 *** | 1.0 | 1.0 | 1.0 | 1.0 | 1.0 | 1.0 | |
| | Sodium hydroxide | adequate amount | adequate amount | adequate amount | adequate amount | adequate amount | adequate amount | |
| | LPG | 7.0 | 7.0 | 7.0 | 7.0 | 7.0 | 7.0 | |
| | Purified water | balance | balance | balance | balance | balance | balance | |
| pH | | 4.5 | 4.5 | 4.5 | 4.5 | 4.5 | 4.5 | |
| Softness | After 3 hrs. | ◎ | ○ | ○ | △ | △ | △ | △ |
| | After 12 hrs. | ◎ | ○ | ○ | △ | × | × | × |
| | After a shampoo and drying with a drier | ○ | ○ | ○ | × | × | × | × |

Note: *: A: Ion Treatment; B: Steam Ladies Iron

**: Equivalent in mole with 10.0% of glycolic acid

***: Foaming agent (polyoxyethylene-sec-tetradecyl ether (9E.O.), produced by Nippon Shokubai Kagaku Kogyo Co., Ltd.)

Standard of Evaluation:

◎ ... Much softer than the comparative hair bundle.

○ ... Softer than the comparative hair bundle.

△ ... Slightly softer than the comparative hair bundle.

× ... No difference from the comparative hair bundle in softness is felt.

As is apparent from the results in Table 2, the hair treated by the method of the present invention has greatly improved and long-lasting softness.

EXAMPLE 7

Hair of a Japanese was shampooed twice and dried, and a bundle weighing 5 g was prepared therefrom.

A hair foam was prepared from 3.0% of 2-naphthalenesulfonic acid, 1.0% of Softanol 90, an adequate amount of sodium hydroxide, 7.0% of LPG, and purified water (the balance). Two grams of the foam were applied to the hair bundle and thoroughly spread throughout the bundle, and the hair was combed for 5 minutes while carefully applying steam all over the bundle by the use of a commercially available hand steamer Ion Treatment.

The thus treated hair bundle was allowed to stand at 20°C and 65% RH for 12 hours and then given a shampoo, followed by drying with a dryer. The bouncy and firmness of the hair bundle was organoleptically examined after 3 hours' standing, after 12 hours' standing, and after 12 hours' standing and a shampoo and drying and evaluated in comparison with a hair bundle having been treated with neither the foam nor steam. The standard of evaluation is shown below. The results obtained are shown in Table 3.

EXAMPLE 8

The same procedure as in Example 7 was repeated, except for replacing 2-naphthalenesulfonic acid with 4.4% of hydroxymethoxybenzophenonesulfonic acid (equivalent in mole to 3.0% of 2-naphthalenesulfonic acid). The results obtained are shown in Table 3.

EXAMPLE 9

The same procedure as in Example 7 was repeated, except for replacing 2-naphthalenesulfonic acid with 3.9% of guaiazulenesulfonic acid (equivalent in mole to 3.0% of 2-naphthalenesulfonic acid) and replacing Ion Treatment with another commercially available hand steamer Steam Ladies Iron. The results of the organoleptic test are shown in Table 3.

COMPARATIVE EXAMPLE 9

The same procedure as in Example 7 was repeated, except that no steam was applied to the hair bundle. The results obtained are shown in Table 3.

COMPARATIVE EXAMPLE 10

The same procedure as in Example 8 was repeated, except that no steam was applied to the hair bundle. The results obtained are shown in Table 3.

COMPARATIVE EXAMPLE 11

The same procedure as in Example 9 was repeated, except that no steam was applied to the hair bundle. The results obtained are shown in Table 3.

COMPARATIVE EXAMPLE 12

The same procedure as in Example 7 was repeated, except that no foam was applied to the hair bundle. The results obtained are shown in Table 3.

TABLE 3

| | | Example | | | Compara. Example | | | |
|---|---|---|---|---|---|---|---|---|
| | | 7 | 8 | 9 | 9 | 10 | 11 | 12 |
| Hand steamer * | | A | A | B | None | None | None | A |
| Composition of Foam (%) | 2-Naphthalene-sulfonic acid | 3.0 | — | — | 3.0 | — | — | not applied |
| | Hydroxymethoxy-benzophenone-sulfonic acid | — | 4.4** | — | — | 4.4** | — | |
| | Guaiazulene-sulfonic acid | — | — | 3.9** | — | — | 3.9** | |
| | Softanol 90 *** | 1.0 | 1.0 | 1.0 | 1.0 | 1.0 | 1.0 | |
| | Sodium hydroxide | adequate amount | adequate amount | adequate amount | adequate amount | adequate amount | adequate amount | |
| | LPG | 7.0 | 7.0 | 7.0 | 7.0 | 7.0 | 7.0 | |
| | Purified water | balance | balance | balance | balance | balance | balance | |
| pH | | 4.5 | 4.5 | 4.5 | 4.5 | 4.5 | 4.5 | |
| Softness | After 3 hrs. | ◎ | ◎ | ○ | △ | △ | △ | × |
| | After 12 hrs. | ◎ | ◎ | ○ | △ | △ | △ | × |
| | After a syampoo and drying with a drier | ○ | ○ | ○ | × | × | × | × |

Note: *: A: Ion Treatment; B: Steam Ladies Iron

**: Equivalent in mole with 3.0% of 2-naphthalene-sulfonic acid

***: Foaming agent (polyoxyethylene-sec-tetradecyl ether (9E.O.), produced by Nippon Shokubai Kagaku Kogyo Co., Ltd.)

Standard of Evaluation:

◎ ... Much more bouncing and firmer than the comparative hair bundle.

○ ... More bounding and firmer than the comparative hair bundle.

△ ... Slightly more bounding and firmer than the comparative hair bundle.

× ... No difference from the comparative hair bundle in bouncy and firmness is felt.

As is apparent from the results in Table 3, the hair treated by the method of the present invention has greatly improved and long-lasting bouncy and firmness.

EXAMPLE 10

Hair of a Japanese was shampooed twice and dried, and a bundle weighing 3 g was prepared therefrom.

A hair foam was prepared from 1.0% of a pentaerythritol-isostearyl glycidyl ether 1-mole adduct, 0.5% of Softanol 90, 7.0% of LPG, and 91.5% of purified water. One gram of the foam was applied to the hair bundle and thoroughly spread throughout the bundle, and the hair was combed for 5 minutes while carefully applying steam all over the bundle by the use of a commercially available hand steamer Ion Treatment.

The thus treated hair bundle was allowed to stand at 20 °C and 65% RH for 12 hours and then given a shampoo, followed by drying with a dryer. The moistness and smoothness of the hair bundle were organoleptically examined after 12 hours' standing and after the shampoo and drying and evaluated according to the respective standard as follows. The results obtained are shown in Table 4.

EXAMPLE 11

The same procedure as in Example 10 was repeated, except for replacing the 1-mole adduct of pentaerythritol-isostearyl glycidyl ether with 1.0% of a 1-mole adduct of pentaerythritol-oleyl glycidyl ether. The results obtained are shown in Table 4.

EXAMPLE 12

The same procedure as in Example 10 was repeated, except for replacing the 1-mole adduct of pentaerythritol-isostearyl glycidyl ether with 1.0% of a pentaerythritol-pentadecyl glycidyl ether adduct and replacing Ion Treatment with another commercially available hand steamer Steam Ladies Iron. The results of the organoleptic test are shown in Table 4.

EXAMPLE 13

The same procedure as in Example 10 was repeated, except for replacing the pentaerythritol-isostearyl glycidyl ether 1-mole adduct with 1.0% of $\alpha$-monoisostearyl glyceryl ether and replacing Ion Treatment with another commercially available hand steamer Steam Kurukuru. The results of the organoleptic test are shown in Table 4.

EXAMPLE 14

The same procedure as in Example 10 was repeated, except for replacing the 1-mole adduct of pentaerythritol-isostearyl glycidyl ether with 1.0% of methylisostearyltrismethylolamide and replacing Ion Treatment with another commercially available hand steamer Steam Kurukuru. The results of the organoleptic test are shown in Table 4.

COMPARATIVE EXAMPLE 13

The same procedure as in Example 10 was repeated, except that no steam was applied to the hair bundle. The results obtained are shown in Table 4.

COMPARATIVE EXAMPLE 14

The same procedure as in Example 11 was repeated, except that no steam was applied to the hair bundle. The results obtained are shown in Table 4.

COMPARATIVE EXAMPLE 15

The same procedure as in Example 12 was repeated, except that no steam was applied to the hair bundle. The results obtained are shown in Table 4.

COMPARATIVE EXAMPLE 16

The same procedure as in Example 13 was repeated, except that no foam was applied to the hair bundle. The results obtained are shown in Table 4.

COMPARATIVE EXAMPLE 17

The same procedure as in Example 14 was repeated, except that no steam was applied to the hair bundle. The results obtained are shown in Table 4.

COMPARATIVE EXAMPLE 18

The same procedure as in Example 13 was repeated, except that no foam was applied to the hair bundle. The results obtained are shown in Table 4.

TABLE 4

| | | Example | | | | | Compara. Example | | | | | |
|---|---|---|---|---|---|---|---|---|---|---|---|---|
| | | 10 | 11 | 12 | 13 | 14 | 13 | 14 | 15 | 16 | 17 | 18 |
| Hand steamer * | | A | A | B | C | C | None | None | None | None | None | C |
| Composition of Foam (%) | Pentaerythritol-isostearyl glycidyl ether 1-mole adduct | 1.0 | – | – | – | – | 1.0 | – | – | – | – | not applied |
| | Pentaerythritol-oleyl glycidyl ether adduct | – | 1.0 | – | – | – | – | 1.0 | – | – | – | |
| | Pentaerythritol-penta decyl glycidyl ether adduct | – | – | 1.0 | – | – | – | – | 1.0 | – | – | |
| | α-Monoisostearyl glycidyl ether | – | – | – | 1.0 | – | – | – | – | 1.0 | – | |
| | Methylisostearyl-trismethylolamide | – | – | – | – | 1.0 | – | – | – | – | 1.0 | |
| | Softanol 90 *** | 0.5 | 0.5 | 0.5 | 0.5 | 0.5 | 0.5 | 0.5 | 0.5 | 0.5 | 0.5 | |
| | LPG | 7.0 | 7.0 | 7.0 | 7.0 | 7.0 | 7.0 | 7.0 | 7.0 | 7.0 | 7.0 | |
| | Purified water | 91.5 | 91.5 | 91.5 | 91.5 | 91.5 | 91.5 | 91.5 | 91.5 | 91.5 | 91.5 | |
| Moistness | After 12 hrs. | ◎ | ◎ | ◎ | ◎ | ◎ | ○ | ○ | ○ | ○ | ○ | |
| | After a Shampoo and drying with a drier | ◎ | ◎ | ◎ | ○ | ○ | ○ | △ | △ | △ | △ | × |
| Smoothness | After 12 hrs. | ○ | ○ | ○ | ○ | ○ | △ | △ | △ | △ | △ | × |
| | After a shampoo and drying with a drier | ○ | ○ | ○ | ○ | ○ | × | × | × | × | × | × |

Note: *: A: Ion Treatment; B: Steam Ladies Iron;

C: Steam Kurukuru

**: Foaming agent

(polyoxyethylene-sec-tetradecyl ether

(9E.O.), produced by Nippon Shokubai Kagaku Kogyo Co., Ltd.)

Standard of Evaluation of Moistness:

◎ ... Very moist

○ ... Moist

△ ... Slightly moist

× ... Not moist

Standard of Evaluation of Smoothness:

◎ ... Very smooth

○ ... Smooth

△ ... Slightly smooth

× ... Not smooth

As is apparent from the results in Table 4, the hair treated by the method of the present invention has greatly improved moistness and smoothness and retains the improved moistness even after a shampoo.

Many other variations and modifications of the present invention will be apparent to those skilled in the art without departing from the spirit and scope thereof. The above-described embodiments are therefore intended to be merely exemplary, and all such variations and modifications are intended to be included within the scope of the present invention as defined in the appended claims.

## Claims

1. A method of hair treatment comprising applying a hair treating preparation to hair and treating the hair with steam either simultaneously with or after the application of the hair treating preparation, in which said hair treating preparation comprises a compound represented by formula (I):

$$R^2-(OCH_2\overset{\overset{\displaystyle R^1}{|}}{C}H)_n-OH \qquad (I)$$

wherein $R^1$ represents a hydrogen atom or a methyl group; $R^2$ represents an alkyl group having 1 to 5 carbon atoms; and n represents an integer of 2 to 6.

2. A method of hair treatment as claimed in claim 1, wherein said compound represented by formula (I) is a monoalkyl ether of diethylene glycol, dipropylene glycol, triethylene glycol or tetraethylene glycol.

3. A method of hair treatment as claimed in claim 2, wherein said compound represented by formula (I) is diethylene glycol monoethyl ether, diethylene glycol monopropyl ether or diethylene glycol monobutyl ether.

4. A method of hair treatment comprising applying a hair treating preparation to hair and treating the hair with steam

either simultaneously with or after the application of the hair treating preparation, in which said hair treating preparation comprises a compound represented by formula (II) or a salt thereof:

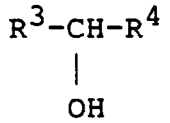

(II)

wherein $R^3$ represents a hydrogen atom or an alkyl group having 1 to 4 carbon atoms; and $R^4$ represents COOH or $SO_3H$.

**5.** A method of hair treatment as claimed in claim 4, wherein said compound represented by formula (II) is glycolic acid, lactic acid, α-hydroxybutyric acid or isethionic acid, and the salt thereof is a sodium salt, a potassium salt, a calcium salt, an ammonium salt, a triethanolamine salt or an organic quaternary ammonium salt.

**6.** A method of hair treatment comprising applying a hair treating preparation to hair and treating the hair with steam either simultaneously with or after the application of the hair treating preparation, in which said hair treating preparation comprises at least one compound selected from the group consisting of a naphthalenesulfonic acid, a benzophenonesulfonic acid, an azulenesulfonic acid, and salts thereof.

**7.** A method of hair treatment as claimed in claim 6, wherein said naphthalenesulfonic acid is represented by formula (III):

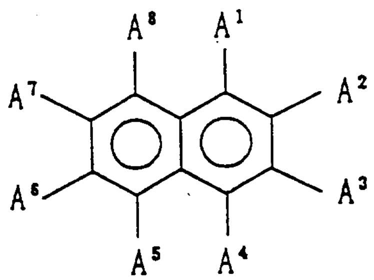

(III)

wherein $A^1$ to $A^8$, which may be the same or different, each represent a sulfo group or a salt thereof, a hydrogen atom, a halogen atom, a hydroxyl group, a nitro group, a carboxyl group, a lower alkoxycarbonyl group, an alkyl group, an alkenyl group, a lower alkoxy group, a formyl group, an acyl group, a substituted or unsubstituted phenylazo group or -NR'R", wherein R' and R", which may be the same or different, each represent a hydrogen atom, a lower alkyl group, a lower alkenyl group, a phenyl group, a benzyl group or an acyl group; and at least one of $A^1$ to $A^8$ is a sulfo group or a salt thereof.

**8.** A method of hair treatment as claimed in claim 7, wherein said naphthalenesulfonic acid is 2-naphthalenesulfonic acid, 1,5-naphthalenedisulfonic acid, 2,6-naphthalenedisulfonic acid, 2,7-naphthalenedisulfonic acid, 1-amino-8-naphthol-3,6-disulfonic acid, 1- naphthalenesulfonic acid, chromotropic acid or a formalin polycondensate of 2-naphthalenesulfonic acid-formalin polycondensate (weight average degree of condensation: 2 to 50).

**9.** A method of hair treatment as claimed in claim 6, wherein said benzophenonesulfonic acid is represented by formula (IV):

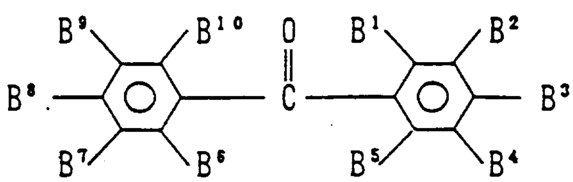

(IV)

wherein $B^1$ to $B^{10}$, which may be the same or different, each represent a sulfo group or a salt thereof, a hydrogen atom, a halogen atom, a hydroxyl group, a carboxyl group, a lower alkyl group, a lower alkenyl group, a lower alkoxy group or an acyl group; and at least one of $B^1$ to $B^{10}$ is a sulfo group or a salt thereof.

**10.** A method of hair treatment comprising applying a hair treating preparation to hair and treating the hair with steam either simultaneously with or after the application of the hair treating preparation, in which said hair treating preparation comprises at least one nonionic amphiphilic compound which contains at least one long-chain branched alkyl group or alkenyl group in the molecule thereof and has an HLB of from 2 to 12, and said nonionic amphiphilic compound or the mixture of said nonionic amphiphilic compound and water maintains a liquid crystal structure at any temperature between 0 and 50 °C.

## Patentansprüche

**1.** Verfahren zur Behandlung von Haar, umfassend

Aufbringen einer Haarbehandlungs-Zubereitung auf Haar und,

gleichzeitig mit oder nach dem Aufbringen der Haarbehandlungs-Zubereitung, Behandeln des Haars mit Dampf, wobei die Haarbehandlungs-Zubereitung eine Verbindung, dargestellt durch die Formel (I), umfaßt:

$$R^2\text{-}(OCH_2\overset{\overset{R^1}{|}}{C}H)_n\text{-}OH \qquad (I)$$

wobei der Rest $R^1$ ein Wasserstoffatom oder ein Methylrest, der Rest $R^2$ ein Alkylrest mit 1 bis 5 Kohlenstoffatomen und n eine ganze Zahl von 2 bis 6 ist.

**2.** Verfahren zur Behandlung von Haar gemäß Anspruch 1, wobei die Verbindung, dargestellt durch Formel (I), ein Monoalkylether von Diethylenglykol, Dipropylenglykol, Triethylenglykol oder Tetraethylenglykol ist.

**3.** Verfahren zur Behandlung von Haar gemäß Anspruch 2, wobei die Verbindung, dargestellt durch Formel (I), Diethylenglykolmonoethylether, Diethylenglykolmonopropylether oder Diethylenglykolmonobutylether ist.

**4.** Verfahren zur Behandlung von Haar, umfassend

Aufbringen einer Haarbehandlungs-Zubereitung auf Haar und,
gleichzeitig mit oder nach dem Aufbringen der Haarbehandlungs-Zubereitung auf das Haar, Behandeln des Haars mit Dampf, wobei die Haarbehandlungs-Zubereitung eine Verbindung, dargestellt durch Formel (II) oder ein Salz davon, umfaßt:

$$R^3\text{-}\underset{\underset{OH}{|}}{C}H\text{-}R^4 \qquad (II)$$

wobei der Rest $R^3$ ein Wasserstoffatom oder ein Alkylrest mit 1 bis 4 Kohlenstoffatomen und der Rest $R^4$ COOH oder $SO_3H$ ist.

**5.** Verfahren zur Behandlung von Haar gemäß Anspruch 4, wobei die Verbindung, dargestellt durch Formel (II), Glykolsäure, Milchsäure, $\alpha$-Hydroxybuttersäure oder Isethionsäure ist und wobei das Salz davon ein Natrium-, Kalium-, Calcium-, Ammonium-, Triethanolaminsalz oder ein quaternäres organisches Ammoniumsalz ist.

**6.** Verfahren zur Behandlung von Haar, umfassend

Aufbringen einer Haarbehandlungs-Zubereitung auf Haar und,

gleichzeitig mit oder nach dem Aufbringen der Haarbehandlungs-Zubereitung, Behandeln des Haars mit Dampf, wobei die Haarbehandlungs-Zubereitung mindestens eine Verbindung, ausgewählt aus der Gruppe bestehend aus, einer Naphthalinsulfonsäure, einer Benzophenonsulfonsäure, einer Azulensulfonsäure und Salzen davon umfaßt.

7. Verfahren zur Behandlung von Haar gemäß Anspruch 6, wobei die Naphthalinsulfonsäure durch die Formel (III) dargestellt wird:

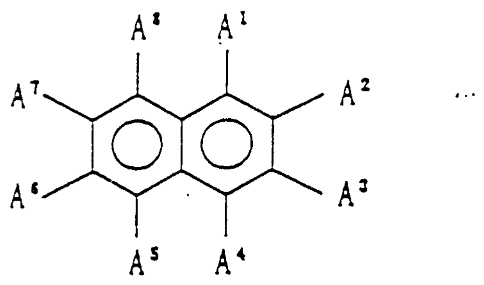

(III)

wobei die Reste $A^1$ bis $A^8$, die gleich oder verschieden sein können, jeweils eine Sulfogruppe oder ein Salz davon, ein Wasserstoffatom, ein Halogenatom, eine Hydroxylgruppe, eine Nitrogruppe, eine Carboxylgruppe, einen Niederalkoxycarbonylrest, einen Alkylrest, einen Alkenylrest, einen Niederalkoxyrest, eine Formylgruppe, einen Acylrest, einen substituierten oder unsubstituierten Phenylazorest oder den Rest -NR'R" darstellen, wobei der Rest R' und R", die gleich oder verschieden sein können, jeweils ein Wasserstoffatom, einen Niederalkylrest, einen Niederalkenylrest, eine Phenylgruppe, eine Benzylgruppe oder einen Acylrest darstellen, und wobei mindestens einer der Reste $A^1$ bis $A^8$ eine Sulfogruppe oder ein Salz davon ist.

8. Verfahren zur Behandlung von Haar gemäß Anspruch 7, wobei die Naphthalinsulfonsäure 2-Naphthalinsulfonsäure, 1,5-Naphthalindisulfonsäure, 2,6-Naphthalindisulfonsäure, 2,7-Naphthalindisulfonsäure, 1-Amino-8-naphthol-3,6-disulfonsäure, 1-Naphthalinsulfonsäure, Chromotropsäure oder ein Formalinpolykondensat von 2-Naphthalinsulfonsäure-formalinpolykondensat (Gewichtsmittelgrad der Kondensation: 2 bis 50) ist.

9. Verfahren zur Behandlung von Haar gemäß Anspruch 6, wobei die Benzophenonsulfonsäure durch die Formel (IV) dargestellt ist:

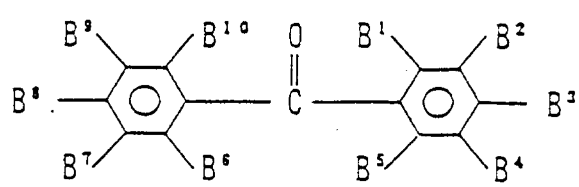

(IV)

wobei die Reste $B^1$ bis $B^{10}$, die gleich oder verschieden sein können, jeweils eine Sulfogruppe oder ein Salz davon, ein Wasserstoffatom, ein Halogenatom, eine Hydroxylgruppe, eine Carboxylgruppe, einen Niederalkylrest, einen Niederalkenylrest, einen Niederalkoxyrest oder einen Acylrest darstellen, und wobei mindestens einer der Reste $B^1$ bis $B^{10}$ eine Sulfogruppe oder ein Salz davon ist.

10. Verfahren zur Behandlung von Haar, umfassend

Aufbringen einer Haarbehandlungs-Zubereitung auf Haar und,

gleichzeitig mit oder nach dem Aufbringen der Haarbehandlungs-Zubereitung, Behandeln des Haars mit Dampf, wobei die Haarbehandlungs-Zubereitung mindestens eine nichtionische amphiphile Verbindung umfaßt, die mindestens einen langkettigen verzweigten Alkylrest oder Alkenylrest in Molekül davon enthält und einen HLB von 2 bis 12 hat und wobei die nichtionische amphiphile Verbindung oder das Gemisch aus der

nichtionischen amphiphilen Verbindung und Wasser eine Flüssigkristallstruktur bei jeder Temperatur zwischen 0 und 50°C beibehält.

## Revendications

1. Procédé de traitement des cheveux comprenant une application, sur les cheveux, d'une préparation de traitement des cheveux et le traitement des cheveux avec de la vapeur, soit simultanément avec l'application de la préparation de traitement des cheveux, soit après, dans lequel ladite préparation de traitement des cheveux comprend un composé représenté par la formule (I) :

$$R^2-(OCH_2\overset{\overset{\displaystyle R^1}{|}}{C}H)_n-OH \qquad (I)$$

dans laquelle $R^1$ représente un atome d'hydrogène ou un groupe méthyle ; $R^2$ représente un groupe alkyle ayant 1 à 5 atomes de carbone ; et n représente un nombre entier de 2 à 6.

2. Procédé de traitement des cheveux tel que revendiqué dans la revendication 1, dans lequel ledit composé représenté par la formule (I) est un éther monoalkylique de diéthylèneglycol, de dipropylèneglycol, de triéthylèneglycol ou de tétraéthylèneglycol.

3. Procédé de traitement des cheveux tel que revendiqué dans la revendication 2, dans lequel ledit composé représenté par la formule (I) est l'éther monoéthylique de diéthylèneglycol, l'éther monopropylique de diéthylèneglycol ou l'éther monobutylique de diéthylèneglycol.

4. Procédé de traitement des cheveux comprenant une application, sur les cheveux, d'une préparation de traitement des cheveux et le traitement des cheveux avec de la vapeur, soit simultanément avec l'application de la préparation de traitement des cheveux, soit après, dans lequel ladite préparation de traitement des cheveux comprend un composé représenté par la formule (II) ou un sel de celui-ci :

$$R^3-\underset{\underset{\displaystyle OH}{|}}{C}H-R^4 \qquad (II)$$

dans laquelle $R^3$ représente un atome d'hydrogène ou un groupe alkyle ayant 1 à 4 atomes de carbone ; et $R^4$ représente COOH ou $SO_3H$.

5. Procédé de traitement des cheveux tel que revendiqué dans la revendication 4, dans lequel ledit composé représenté par la formule (II) est l'acide glycolique, l'acide lactique, l'acide $\alpha$-hydroxybutyrique ou l'acide iséthionique et le sel de celui-ci est un sel de sodium, un sel de potassium, un sel de calcium, un sel d'ammonium, un sel de triéthanolamine ou un sel d'ammonium quaternaire organique.

6. Procédé de traitement des cheveux comprenant une application, sur les cheveux, d'une préparation de traitement des cheveux et le traitement des cheveux avec de la vapeur, soit simultanément avec l'application de la préparation de traitement des cheveux, soit après, dans lequel ladite préparation de traitement des cheveux comprend au moins un composé choisi à partir du groupe constitué d'un acide naphtalène-sulfonique, d'un acide benzophénone-sulfonique, d'un acide azulène-sulfonique et des sels de ceux-ci.

7. Procédé de traitement des cheveux tel que revendiqué dans la revendication 6, dans lequel ledit acide naphtalène-sulfonique est représenté par la formule (III) :

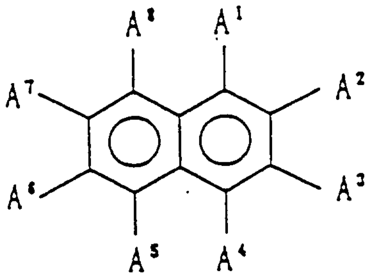

(III)

dans laquelle $A^1$ à $A^8$, qui peuvent être identiques ou différents, représentent chacun un groupe sulfo ou un sel de celui-ci, un atome d'hydrogène, un atome d'halogène, un groupe hydroxyle, un groupe nitro, un groupe carboxyle, un groupe alcoxycarbonyle inférieur, un groupe alkyle, un groupe alcényle, un groupe alcoxy inférieur, un groupe formyle, un groupe acyle, un groupe phénylazo substitué ou non substitué ou -NR'R", dans lequel R' et R", qui peuvent être identiques ou différents, représentent chacun un atome d'hydrogène, un groupe alkyle inférieur, un groupe alcényle inférieur, un groupe phényle, un groupe benzyle, ou un groupe acyle ; et au moins l'un de $A^1$ à $A^8$ est un groupe sulfo ou un sel de celui-ci.

**8.** Procédé de traitement des cheveux tel que revendiqué dans la revendication 7, dans lequel ledit acide naphtalène-sulfonique est l'acide 2-naphtalène-sulfonique, l'acide 1,5-naphtalène-disulfonique, l'acide 2,6-naphtalène-disulfonique, l'acide 2,7-naphtalène-disulfonique, l'acide 1-amino-8-naphtol-3,6-disulfonique, l'acide 1-naphtalène-sulfonique, l'acide chromotropique, ou un polycondensat de Formaline d'un polycondensat d'acide 2-naphtalène-sulfonique et de Formaline (degré de condensation moyen en poids : 2 à 50).

**9.** Procédé de traitement des cheveux tel que revendiqué dans la revendication 6, dans lequel ledit acide benzophénone-sulfonique est représenté par la formule (IV) :

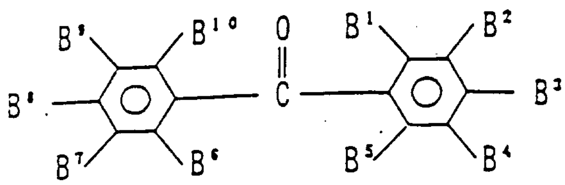

(IV)

dans laquelle $B^1$ à $B^{10}$, qui peuvent être identiques ou différents, représentent chacun un groupe sulfo ou un sel de celui-ci, un atome d'hydrogène, un atome d'halogène, un groupe hydroxyle, un groupe carboxyle, un groupe alkyle inférieur, un groupe alcényle inférieur, un groupe alcoxy inférieur ou un groupe acyle ; et au moins l'un de $B^1$ à $B^{10}$ est un groupe sulfo ou un sel de celui-ci.

**10.** Procédé de traitement des cheveux comprenant une application, sur les cheveux, d'une préparation de traitement des cheveux et le traitement des cheveux avec de la vapeur, soit simultanément avec l'application de la préparation de traitement des cheveux, soit après, dans lequel ladite préparation de traitement des cheveux comprend au moins un composé amphiphile non ionique qui contient au moins un groupe alkyle, ou un groupe alcényle, ramifié à longue chaîne dans la molécule de celui-ci et possède un HLB de 2 à 12, et ledit composé amphiphile non ionique ou le mélange dudit composé amphiphile non ionique et d'eau conserve une structure de cristal liquide à n'importe quelle température entre 0 et 50°C.